# EUROPEAN PATENT SPECIFICATION

(11) **EP 2 231 216 B1**
(45) Date of publication and mention of the grant of the patent: **08.08.2012**
(21) Application number: 08862659.3
(22) Date of filing: 12.12.2008
(51) Int. Cl.: A61L 31/08, A61L 31/16

(54) **DRUG-ELUTING ENDOPROSTHESIS**
ARZNEIMITTEL FREISETZENDE ENDOPROTHESE
ENDOPROTHÈSE ÉLUTRICE DE MÉDICAMENTS

(30) Priority: 14.12.2007 US 13905
(43) Date of publication of application: 29.09.2010
(73) Proprietor: Boston Scientific Scimed, Inc., Maple Grove, MN 55311-1566 (US)
(72) Inventor: CLARKE, John T., Co. Galway (IE); O'CONNOR, Tim, Co. Galway (IE); XU, Yixin, Newton Massachusetts 02462 (US); O'BRIEN, Barry, Galway (IE); ARCAND, Ben, Minneapolis Minnesota 55406 (US); SHIPPY, James Lee, III, Maple Grove Minnesota 55311 (US); ATANASOSKA, Liliana, Edina Minnesota 55436 (US); FLANAGAN, Aiden, Co. Galway (IE); MCMORROW, Dave, Galway (IE); WEBER, Jan, 6228 GJ Maastricht (NL); KREMER, John, Albertville Minnesota 55301 (US); KUEHLING, Michael, 80804 Munich (DE); SEIDEL, Dominique, 80796 Munich (DE)
(74) Representative: Peterreins, Frank
(86) International application number: PCT/US2008/086639
(87) International publication number: WO 2009/079389

(56) References cited:
- WO-A2-2006/108065
- WO-A2-2008/106271
- WO-A2-2008/118606

## Description

### TECHNICAL FIELD

This invention relates to drug-eluting endoprostheses.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with an endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism can include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

The endoprosthesis can carry a drug, such as an antiproliferative, to reduce the likelihood of restenosis, i.e., reclosure of the vessel due to immune reactions by the body at the treatment site. Polymers can be used to control the kinetic drug release ("KDR") from drug-eluting stents ("DES").

WO 2006/108065 A2 discloses drug eluting endoprosthesis wherein bioerodable metals are included as material to form a rate limiting barrier layer to control the drug release from discrete deposits comprising therapeutic agents.

### SUMMARY

A drug-eluting endoprosthesis is disclosed that includes a plurality of discrete deposits and a plurality of overlying layers each overlying one of the plurality of discrete deposits. Each discrete deposit includes one or more therapeutic agents. Each overlying layer includes one or more bioerodible metals. The overlying layers erode in a physiological environment to release the one or more therapeutic agents. In some embodiments, the endoprosthesis can include a body defined by a plurality of struts and define a flow passage therethrough. In some embodiments, at least one of the struts includes the plurality of discrete deposits and the plurality of overlying layers. For example, the endoprosthesis can be a stent.

The overlying layers each can include a bioerodible metal selected from magnesium, zinc, iron, and alloys thereof. In some embodiments, at least two of the overlying layers can include different bioerodible metal compositions. The at least two overlying layers include different bioerodible metal compositions and can each overlie discrete deposits comprising therapeutic agents of different compositions. The therapeutic agents of different compositions can have different release profiles. In some embodiments, the endoprosthesis can include at least a first overlying layer including iron or an alloy thereof and at least a second overlying layer including magnesium or an alloy thereof. In some embodiments, at least two of the overlying layers have different thicknesses. The overlying layers having different thicknesses can each overlie discrete deposits including therapeutic agents of different compositions. The therapeutic agents of different compositions can have different release profiles.

The endoprosthesis can include at least two of the discrete deposits comprise therapeutic agents of different compositions. The therapeutic agents of different compositions can have different release profiles. In some embodiments, the discrete deposits comprise a ceramic or polymeric carrier. In some embodiments, the plurality of discrete deposits are on an abluminal side of the strut.

The body can include a metal selected from the group consisting of stainless steel, platinum enhanced stainless steel, Co-Cr, nitinol, niobium, tantalum, titanium, iridium, platinum, and combinations and alloys thereof. For example, the at least one strut can include a metal selected from the group consisting of stainless steel, platinum enhanced stainless steel, Co-Cr, nitinol, niobium, tantalum, titanium, iridium, platinum, and combinations and alloys thereof.

The body, in some embodiments, can include a primer layer. For example, the at least one strut comprises a primer layer.

In another aspect, a drug-eluting endoprosthesis includes a bioerodible metal portion and a therapeutic agent. The bioerodible metal portion includes at least two bioerodible metal regions having different electronegativities, which are in electrical contact with each other. The bioerodible metal erodes in a physiological environment to release the therapeutic agent.

In some embodiments, the bioerodible metal overlies the therapeutic agent. In some embodiments, the bioerodible metal can include magnesium, zinc, iron, or an alloy thereof. In some embodiments, the bioerodible metal portion can include at least two bioerodible metal regions have different bioerodible metal compositions. In some embodiments, the first region of bioerodible metal can include an embedded therapeutic agent and the second region of bioerodible metal can be at the surface of the endoprosthesis. For example, the first region of bioerodible metal can include iron or an alloy thereof and the second region of bioerodible metal can include magnesium or an alloy thereof.

In some embodiments, the bioerodible metal can include a non-bioerodible portion. For example, the non-bioerodible portion can include stainless steel, platinum enhanced stainless steel, Co-Cr, nitinol, or combinations thereof. In some embodiments, the non-bioerodible portion can include a plurality of pores that contain the therapeutic agent, and the bioerodible metal portion can overlie a surface of the non-bioerodible metal to entrap the therapeutic agent within the pores. In some embodiments, the endoprosthesis can include a first therapeutic agent within the pores and a second therapeutic agent embedded within the bioerodible metal portion. In some embodiments, the therapeutic agent can be provided on a surface of the non-bioerodible portion and the bioerodible metal portion can overlie the therapeutic agent. In some embodiments, the endoprosthesis can include a non-bioerodible scaffolding into which the bioerodible metal and the therapeutic agent are incorporated such that erosion of at least some of the bioerodible metal within the scaffolding releases the therapeutic agent.

In some embodiments, the endoprosthesis can include a plurality of discrete deposits of the therapeutic agent. In some embodiments, the endoprosthesis can include a first therapeutic agent and a second therapeutic agent. The first therapeutic agent and the second therapeutic agent can have different release profiles. In some embodiments, the therapeutic agent can be provided within a ceramic or polymeric carrier, and the ceramic or polymeric carrier can be embedded within the endoprosthesis. In some embodiments, the endoprosthesis can be polymer free. In some embodiments, the endoprosthesis can include a surface deposited therapeutic agent adapted for a burst release of the therapeutic agent upon implantation of the endoprosthesis in a patient's body.

In some embodiments, the endoprosthesis can be a stent.

Methods for forming the drug-eluting endoprostheses are also described. In one aspect, a method includes depositing a plurality of discrete deposits onto at least on strut of body so that the each discrete deposit includes at least one therapeutic agent and depositing a plurality of overlying layers so that each of the plurality of overlying layers overlies one discrete deposit. The plurality of overlying layers each include a bioerodible metal. In some embodiments, the body including a plurality of interconnected struts and defines a flow passage therethrough.

In some embodiments, the plurality of discrete deposits are deposited on a abluminal side of the at least one strut. At least two of the overlying layers can each include different bioerodible metal compositions, different thicknesses, or a combination thereof so that the underlying discrete deposits have different release profiles when the endoprosthesis is implanted within a physiological environment. The at least two overlying layers can overlie discrete deposits each having different therapeutic agent compositions.

In another aspect, a method includes incorporating a therapeutic agent in an endoprosthesis, or precursor thereof, wherein the endoprosthesis, or precursor thereof, includes at least two bioerodible metal regions having different electronegativities and the bioerodible metal erodes in a physiological environment to release the therapeutic agent.

In some embodiments, incorporating the therapeutic agent in the endoprosthesis can include depositing a bioerodible metal onto an endoprosthesis, or precursor thereof, to form the bioerodible metal portion such that the bioerodible metal entraps at least a portion of the therapeutic agent within the drug-eluting endoprosthesis. For example, the therapeutic agent and the bioerodible metal can be concurrently deposited.

In some embodiments, the method can further include depositing a non-bioerodible metal concurrently with depositing the therapeutic agent. For example, the bioerodible metal, the non-bioerodible metal, and the therapeutic agent can be concurrently deposited.

In some embodiments, the bioerodible metal, the non-bioerodible metal and/or the therapeutic agent can deposited using cold gas dynamic spraying techniques. In some embodiments, the therapeutic agent can be deposited while in a bioerodible carrier (e.g., a plastic or ceramic bioerodible carrier).

In some embodiments, the endoprosthesis, or precursor thereof, can include a plurality of pores and the therapeutic agent can be deposited within the plurality of pores.

The details of one or more embodiments are set forth in the accompanying drawings and the description below. Other features, objects, and advantages will be apparent from the description and drawings, and from the claims.

### DESCRIPTION OF DRAWINGS

Fig. 1 is a perspective view of an example of an expanded stent.
Figs. 2A - 2D show various examples of stent strut cross-sections having drug-spots masked with bioerodible metal.
Fig. 3 depicts a section of a stent strut having bands of drugs masked with bands of degradable metal.
Fig. 4 depicts a magnified image of drug-eluting spots formed on a stent.
Figs. 5A - E show various examples of how bioerodible metal may be layered over the therapeutic agent in a drug eluting stent.
Figs. 6A - 6C show various examples of arrangements where the stent includes a second bioerodible metal.
Figs. 7A - 7D show an arrangement where the stent includes a network of non-bioerodible metal that includes the therapeutic agent and the bioerodible metal.
Figs. 8A- 8C depict an example of how a drug-eluting stent can break down in a physiological environment to release the therapeutic agent.
Fig. 9 depicts an exemplary therapeutic agent release profile.
Fig. 10 depicts a method of producing a drug-eluting stent coating including bioerodible phases and therapeutic agent phases.

Like reference symbols in the various drawings indicate like elements.

### DETAILED DESCRIPTION

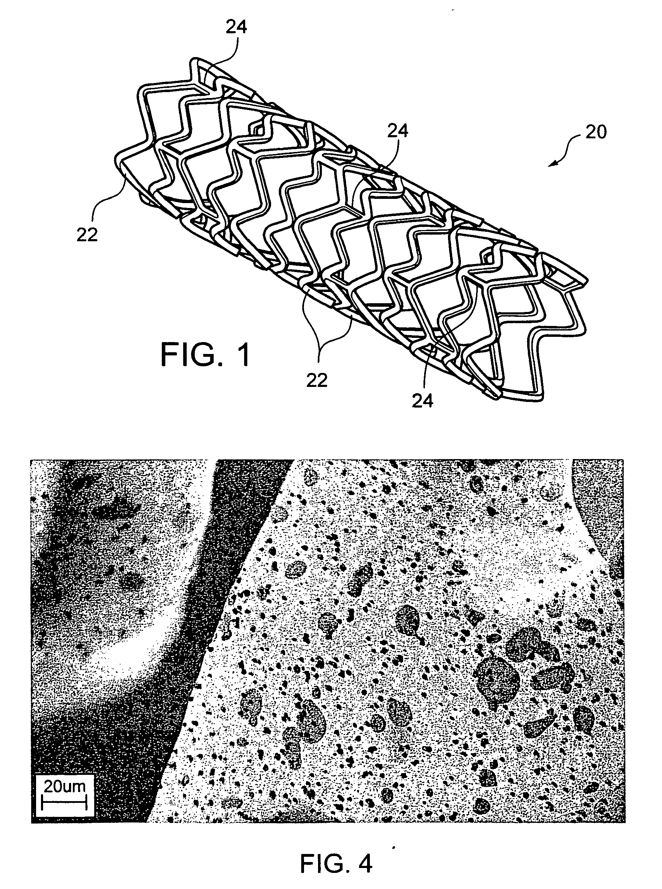

Referring to Fig. 1, a stent 20 can have the form of a tubular member defined by a plurality of bands 22 and a plurality of connectors 24 that extend between and connect adjacent bands. During use, bands 22 can be expanded from an initial, small diameter to a larger diameter to contact stent 20 against a wall of a vessel, thereby maintaining the patency of the vessel. Connectors 24 can provide stent 20 with flexibility and conformability that allow the stent to adapt to the contours of the vessel.

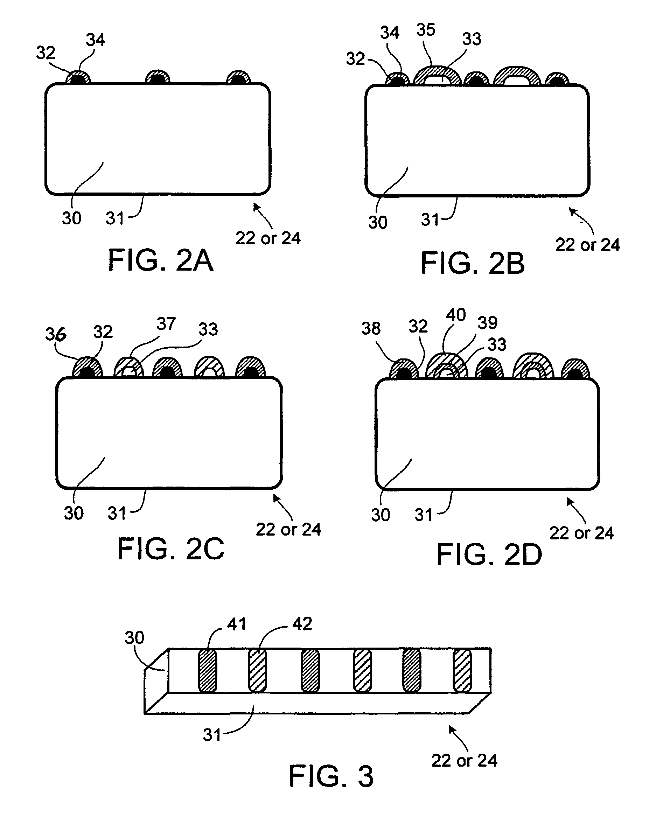

FIGS. 2A - 2D depict various examples of cross-sections of drug-eluting stents that include discrete deposits of one or more therapeutic agents and discrete deposits of one or more bioerodible metals each overlying a discrete deposit of therapeutic agent. The bioerodible metals erode in a physiological environment to release the underlying therapeutic agent. The erosion of each discrete deposit of the bioerodible metal can control the kinetic drug release of the underlying therapeutic agent. Each of the examples shown includes a structural member 30. The structural member 30 can also include a thin primer layer 31 that can act as a tie layer to improve the adhesion of the deposited therapeutic agents and portions of the discrete deposits of bioerodible metal.

As depicted in FIGS. 2A-2D and as shown in FIG. 4, the stent can include multiple drug-eluting deposits across the width or length of a stent strut. In other embodiments, such as shown in FIG. 3, each drug-eluting deposit can be in the form of bands 41 and 42 extending across one side of a stent strut. In other embodiments, a deposit can encircle a stent strut. Although only shown on a single side of the stent strut, the drug-eluting deposits can be multiple sides of one or more stent struts. In some embodiments, the drug-eluting deposits are deposited only on an abluminal side of the surface of a structural member 30 of the stent. The drug-eluting deposits can be ordered or non-ordered, in-line or out of phase, have equal or different dimensions, and can be homogeneous over the stent or non homogeneous (e.g., concentrated at the stent-ends or clustered).

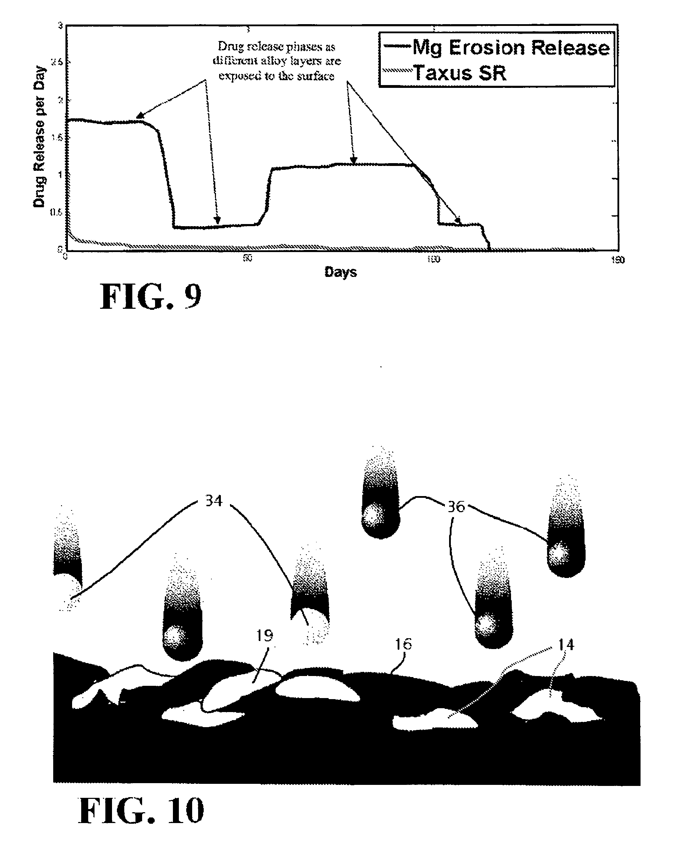

The one or more therapeutic agents are applied in discrete deposits. The therapeutic agents can be deposited as a pure therapeutic agent or with inactive ingredients. The therapeutic agent deposits, in some embodiments, can include a polymer or ceramic matrix. In some embodiments, the stent is polymer-free. In some embodiments, the plurality of different discrete deposits of therapeutic agents can have therapeutic agents of different compositions. For example, a stent could be designed to release one or more therapeutic agents in a physiological environment, with the different therapeutic agents having different release profiles. An example of a release profile is shown in Fig. 9 and discussed below. The size of the therapeutic agent deposits can impact the release profiles for each therapeutic agent.

The overlying layers of one or more bioerodible metals can include bioerodible metals selected from, for example, magnesium, zinc, iron, and alloys thereof. For example, the discrete deposit of bioerodible metal can include a bioerodible iron alloy that includes up to twenty percent manganese, up to 10 percent silver, and up to five percent carbon. A discrete deposit of bioerodible metal can also include a bioerodible magnesium alloy that can contain up to 10% of a mix of the rare metal species from the following: lanthanum, neodymium, holmium, erbium, gadolinium, cerium, dysprosium, praseodymium, promethium, samarium, europium, terbium, thulium, ytterbium, lutetium, actinium, thorium, einsteinium, americium, protactinium, californium, uranium, neptunium, plutonium, curium, berkelium, fermium, mendelevium, nobelium and lawrencium. In some embodiments, a bioerodible magnesium alloy can includes up to nine percent aluminum, up to five percent rare earth metals, up to five percent zirconium, up to five percent lithium, up to five percent manganese, up to ten percent silver, up to five percent chromium, up to five percent silicon, up to five percent tin, up to six percent yttrium, and up to ten percent zinc. Suitable magnesium bioerodible alloys include ZK31, which includes three percent zinc and one percent zirconium; ZK61, which includes six percent zinc and one percent zirconium; AZ31, which includes three percent aluminum and one percent zinc; AZ91, which includes nine percent aluminum and one percent zinc; WE43, which includes four percent yttrium and three percent rare earth metals, and WE54, which includes five percent yttrium and four percent rare earth metals. The release profile of the stent can be controlled by the composition of the bioerodible metal and/or the thickness of each discrete deposit.

The rate of release of each deposit of therapeutic agent is controlled by the composition and thickness of the overlying layer of bioerodible metal. The thickness of the bioerodible metal can range from between 10 nanometers and 200 micrometers. For example, a bioerodible magnesium alloy can bioerode at a rate of between 0.2µm to 1.7 µm per day, depending on the impurities. Accordingly, a 0.1 micrometer thick layer of bioerodible magnesium can be tailored to bioerode in 4 to 12 hours to result in a drug elution 'burst' effect. A 1 micrometer thick layer of bioerodible magnesium can bioerode in 12 hours to 5 days to result in a more prolonged release. For much longer release, an bioerodible iron layer will bioerode at a rate of about 0.1 µm per day, depending on impurities, so a 1 micrometer thick layer of bioerodible iron will bioerode in about 10 days. The thicknesses of different overlying layers can be different. For example, by having steadily varying thicknesses, a stent can have a steady release of therapeutic agents with gradual increases and decreases when implanted in a physiological environment. In other embodiments, the thicknesses of the bioerodible metal overlying layers can result in bursts of therapeutic agent separated in time. In some embodiments, the thickness of different layers can differ by a factor of 1:20,000. For example, a stent can be designed to have a first burst of a first therapeutic agent a few hours after implantation and a second burst of a second therapeutic agent after a month by having one set of drug-eluting deposits of the second therapeutic agent having an overlying coating having thickness that is 200 times thicker than a set of drug-eluting deposits of the first therapeutic agent when using the same bioerodible metal. In some embodiments, the presence of two or more bioerodible metals can result in a galvanic couple when implanted within a physiological environment, which can impact the erosion characteristics of the bioerodible metals.

The structural member 30 of the stent 20 can include a metal and/or a polymer. In some embodiments, the structural member 30 can include a stainless steel alloy, a platinum enhanced stainless steel alloy, a cobalt-chromium alloy, a nickel-titanium alloy, or a combination thereof. In some embodiments, the structural member 30 can include a biostable or bioerodible polymer. For example, the structural member can include poly (lactic acid) ("PLA"). In some embodiments, the structural member 30 can be a biodegradable metal and the stent 20 can be completely bioerodible. In some embodiments, the structural member 30 can include one or more polymers, ceramics, and other structural materials. In some embodiments, the structural member can include one or more radiopaque materials, either as a layer of the structural member or as an alloying element. In some embodiments, an outer layer of the structural member 30 can include a passivizing layer. Passivizing layers can include oxides, nitrides, and carboxides. For example, the structural member can include a passivizing layer of iridium oxide.

The discrete deposits of therapeutic agent can be applied directly to the structural member 30. In some embodiments, the structural member 30 can have a roughened outer surface to increase adhesion of the therapeutic agent to the structural member 30, as well as the adhesion of portions of the overlying layers of bioerodible metal. The structural member 30 can include also include a primer layer 31 to increase the adhesion of the drug-eluting deposits to the structural member 30. For example, the primer layer can be the same bioerodible metal overlying the therapeutic agent. The primer layer can put applied in a non-drug friendly environment (e.g., using Physical Vapor Depositon at high temperatures) to assure good adhesion. The bioerodible metal overlying the therapeutic agent can then be applied afterwards in a drug-friendly deposition process and the use of the same material can facilitate adhesion between the bioerodible metal overlying the therapeutic agent and the primer layer. In other embodiments, the primer layer 31 can be titanium. In some embodiments, the primer layer 31 can also serve as a radiopaque layer and/or as a passivizing layer, e.g., an outer layer of iridium oxide with a nanostructure surface.

FIG. 2A depicts a first embodiment of a stent including therapeutic agent deposits 32 and overlying layers 34 each overlying a discrete deposit 32 of therapeutic agent. For example, the overlying layers 34 can include magnesium and can have a thickness of 1 micrometer. In some embodiments, each drug-eluting deposit can be approximately constant. In other embodiments, such as shown in FIGS. 2B-2D, the drug-eluting deposits can vary in drug composition, in drug deposit size, in bioerodible metal composition, and/or in bioerodible metal thickness.

FIG. 2B includes therapeutic agent deposits 32 including a first therapeutic agent and overlying layers 34 including a first bioerodible metal each overlying one of therapeutic agent deposits 32 and therapeutic agent deposits 33 including a second therapeutic agent and overlying layers 35 of the first bioerodible metal each overlying one of the therapeutic agent deposits 33. Overlying layers 35 each have a thickness greater than the thickness of each of the overlying layers 34, accordingly the first therapeutic agent is released into a physiological environment sooner after implantation than the second therapeutic agent.

FIG. 2C depicts discrete therapeutic agent deposits 32 and 33 of different therapeutic agents each having overlying layers 36 and 37, respectively, of different bioerodible metals. Overlying layers 36 and 37 have the same thickness. The different bioerodible metals can have different erosion characteristics. For example, the overlying layer 37 can include magnesium and overlying layer 38 can include iron. Iron has a slower erosion rate than magnesium, accordingly the therapeutic agent of deposit 32 is released into a physiological environment sooner after implantation than the therapeutic agent of deposit 33. In other embodiments, overlying layers 36 and 37 can have different thicknesses and different compositions.

The drug-eluting deposits can also each have multiple overlying layers of different bioerodible metals, which can further control the release of the underlying therapeutic agent. As shown in FIG. 2D, a first therapeutic agent deposit 32 can include a single overlying layer 38 of a first bioerodible metal and deposits 33 of a second therapeutic agent can having both a first layer 39 of the first bioerodible metal overlying deposit 33 and a second layer 40 of a second bioerodible metal overlying the first layer 39. For example, the first bioerodible metal can be magnesium and the second bioerodible metal can be iron. The presence of the second layer 40 can delay the erosion of the first layer 39, which ultimately results in the delay of the release of the second therapeutic agent of deposit 33.

The plurality of discrete deposits of one or more therapeutic agents can be deposited conventional printing techniques, such as dipping, spraying, roll coating, and ink-jetting. Some processes may include masking techniques to achieve the desired pattern. For example, a pattern of one or more therapeutic agent deposits can be deposited on the abluminal surface of a stent 20 by ink jet printing techniques. The discrete deposits can also be patterned by the use of masking techniques. Cold Gas Dynamic Spray ("CGDS") can also be used to deposit some forms of therapeutic agent deposits. CGDS is described below. In some embodiments, the therapeutic agent can be deposited within a polymer or ceramic matrix to facilitate the deposition process. In some embodiments, the therapeutic agent deposits can include other additives and/or fillers.

The overlying layers of bioerodible metal can be deposited over the discrete therapeutic agent deposits by physical laser deposition ("PLD"), CGDS, and other room temperature processes. Masking of the stent can allow for the selective encapsulation of the discrete therapeutic agent deposits. The stent can be masked with a slotted tube including apertures matching the shape of the intended pattern. The slotted tube can be a wire grid. The primer layer 31 can also be deposited by a CGDS process.

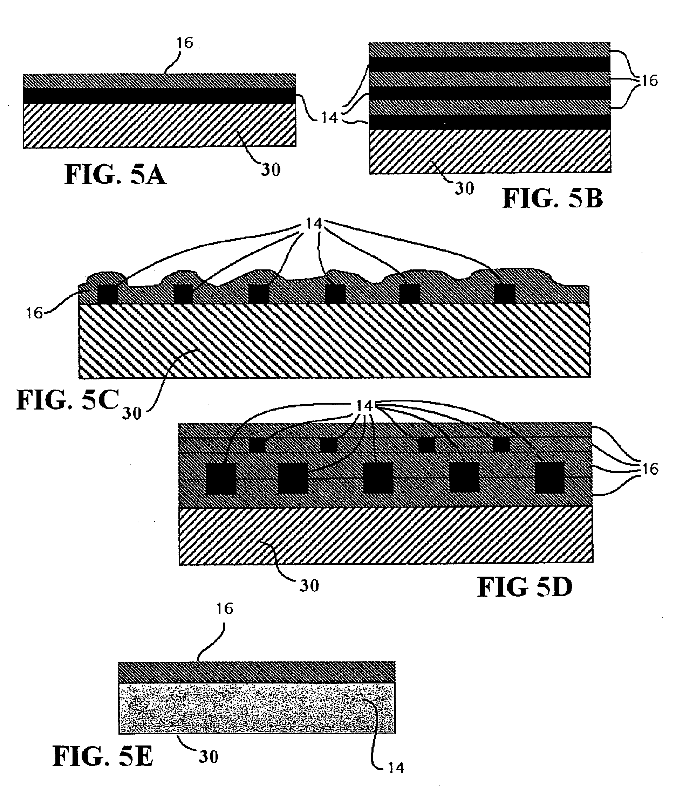

Figs. 5A - 5E depict various examples of drug-eluting stents according to another embodiment. As shown in Fig. 5A, the therapeutic agent 14 can be in the form of a continuous coating over the metal structural member 30 and bioerodible metal 16 can be in the form of a layer overlying the therapeutic agent 14. In some embodiments, the therapeutic agent coating 16 can include multiple therapeutic agents. Although Fig. 5A depicts an even coat, in practice the coating can be irregular.

As shown in Fig. 5C, the therapeutic agent can be in the form of discrete deposits on the metal structural member 30, and the bioerodible metal 16 can be in the form of a layer overlying the deposits of therapeutic agent 14 and the metal structural member 30. As shown in Fig. 5C, the bioerodible metal top coating can be irregular. An irregular top coating can result in a broader time distribution of the release of the therapeutic agent. The different discrete deposits of therapeutic agent can be the same or different and provided separately or in combinations with each other. Although the various deposits of therapeutic agents 14 shown in Fig. 5C are approximately the same size, each deposits 14 may vary in size and shape.

As those shown in Figs. 5B and 5D, the stent 10 can include a plurality of layers of and/or deposits of therapeutic agent and a plurality of layers of bioerodible metal 16. Fig. 5B depicts a drug-eluting stent having multiple coatings of therapeutic agent(s) and of bioerodible metals. The quantity and/or composition of each therapeutic agent layer can be varied. Similarly, the thickness and/or composition of the bioerodible metal layers 16 may also vary. For example, each layer of bioerodible metal could include a different alloy, each alloy having a different erosion rate. The thickness of each bioerodible metal layer 16 will also impact the timing and rate of release of the therapeutic agent(s). Fig. 5D depicts an arrangement containing a plurality of discrete deposits of therapeutic agent(s) 14 between various layers of bioerodible metal 16. Again, each discrete deposit of therapeutic agent 14 and layer of bioerodible metal 16 may vary in size, shape, and/or composition, impacting the release schedule of the therapeutic agent(s) 14.

Fig. 5E depicts an arrangement where the metal structural member 30 includes pores. The therapeutic agent 14 resides within the pores of the metal structural member 30. The bioerodible metal 16 overlies the surface of the metal structural member 30 to prevent the diffusion of the therapeutic agent out of the stent 10 until the bioerodible metal 16 erodes in a physiological environment. The pores can be micropores and/or nanopores. In other embodiments, a substrate may include larger indentations and/or grooves for receiving therapeutic agents. In embodiments where one or more therapeutic agents are deposited within pores, the drug release schedule is controlled both by the erosion rate of the bioerodible metal but also by the slower diffusion of the therapeutic agent out of the porous surface. The pore sizes will impact the rate of diffusion. The therapeutic agent deposited within the pores can be a pure therapeutic agent, a mixture of therapeutic agents, or a mixture that includes inactive ingredients. The therapeutic agent could also be deposited within the pores with a bioerodible polymer that also impacts the kinetic drug release. The therapeutic agent could also be deposited within the pores in the form of a ceramic. This feature of having a therapeutic agent deposited within pores in the surface of a structural member can also be combined with the other features discussed herein.

The layers of bioerodible metal 16 shown in Figs. 5B and 5D can have two or more compositions. By including different layers of different compositions, the erosion characteristics of the layers can be controlled to produce a stent having a desired therapeutic agent release profile. For example, an outer bioerodible metal layer could include pure magnesium and a second bioerodible metal layer could include an alloy of magnesium designed to reduce the erosion rate. Alternatively, an outer bioerodible metal layer could include zinc or an alloy thereof and a second bioerodible metal layer could include iron or an alloy thereof.

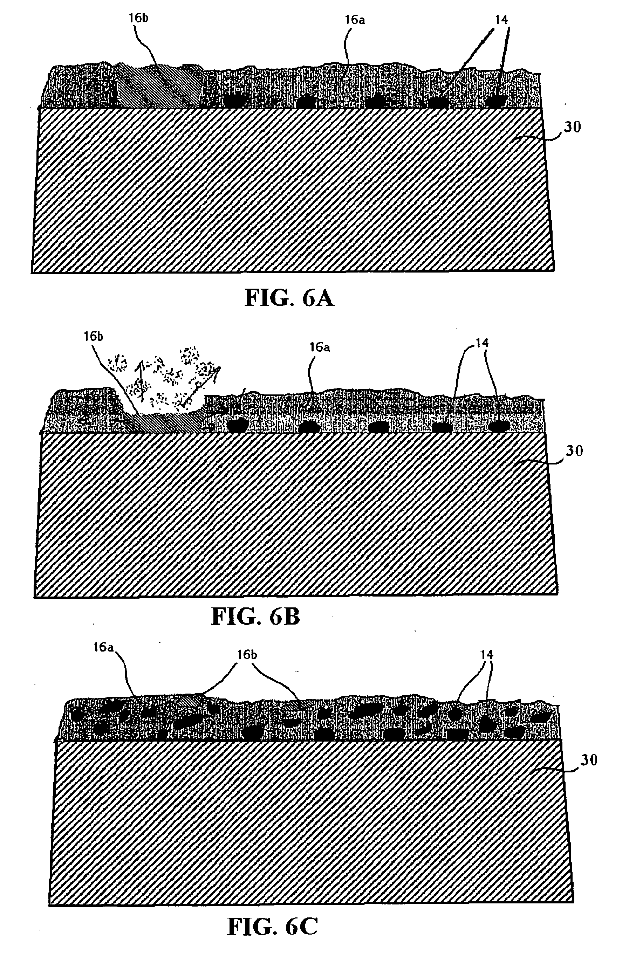

As shown in Figs. 6A- 6C, the stent 20 can include a first bioerodible metal 16a and a second bioerodible metal 16b where the first and second bioerodible metals are in electrical contact with each other. The first and second bioerodible metals can have different electronegativities. As shown in Figs. 6A and 6B, the second bioerodible metal 16b can be less electronegative than the first bioerodible metal 16a. The less electronegative bioerodible metal can be situated in the stent to be exposed to a physiological environment when implanted into a patient's body. As shown in Fig. 6B, the second bioerodible metal 16b, having the lower electronegativity and being in electrical contact with the first bioerodible metal 16a, can erode preferentially relative to the first bioerodible metal 16a. The less electronegative bioerodible metal can protect the more electronegative bioerodible metal by acting as a galvanic anode. Electrons can flow from the less electronegative bioerodible metal to the more electronegative bioerodible metal to slow down or prevent the corrosion reaction of the more electronegative bioerodible metal until the second bioerodible metal is completely eroded. For example, the first bioerodible metal 16a can be iron or an alloy thereof and the second bioerodible metal 16b can be magnesium or an alloy thereof. In some embodiments, zinc or an alloy thereof could act as either the first or second bioerodible metal 16a or 16b, as zinc is less electronegative than iron but more electronegative than magnesium. In some embodiments, a stent could include regions of magnesium or alloys thereof, zinc or alloys thereof, and iron or alloys thereof, with any or all of the regions controlling the release of therapeutic agents 14.

As shown in Fig. 6C, the second bioerodible metal 16b can be included as a deposit within a matrix of the first bioerodible metal 16a. The second bioerodible metal 16b could also be in the form of deposited strips or dots on the outside of the first bioerodible metal 16a. In other arrangements, not shown, the stent can form the electrical connection through the metal structural member 30 or other portions of the stent 12. For example, the second bioerodible metal 16b could be included as a plug into a stent strut at various locations.

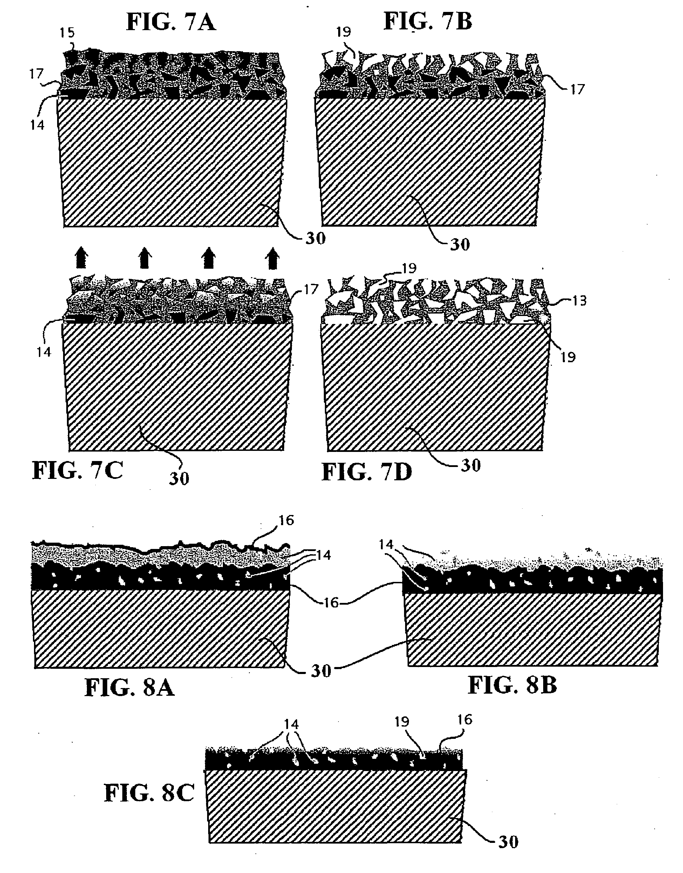

Figs. 7A - 7D show an arrangement where the stent includes a network of non-bioerodible metal that includes the therapeutic agent and the bioerodible metal. As shown in Fig. 7A, the stent 20 can include a metal network 17 and therapeutic agent deposited within the metal network 17. The metal network 17 includes a network of non-bioerodible metal and bioerodible metal portions. As shown in Fig. 7A, the surface of the stent, prior to insertion into the body, can include surface pores 19 including therapeutic agent 14. These surface deposits of therapeutic agent 14 can elude almost immediately out of the metal network when placed in a physiological environment, resulting in the structure of Fig. 7B. This quick elusion is sometimes referred to as a "burst release" of therapeutic agent 14. Then, while situated in a physiological environment, the bioerodible metal can erode out of the metal network 17, as shown in Fig. 7C. As the bioerodible metal erodes from the metal network 17, additional deposits of therapeutic agent 14 can be released into the physiological environment. After all of the bioerodible metal has eroded and all of the therapeutic agent 14 has been released, the structure shown in Fig. 7D can remain. Non-limiting examples of suitable non-bioerodible metals for inclusion in the network include stainless steels, platinum enhanced stainless steels, cobalt-chromium alloys, nickel-titanium alloys, tantalum, titanium, niobium, iridium, platinum, gold, and alloys or ceramics thereof. Non-limiting examples of ceramics can include oxides, carbides, and nitrides of metals such as zirconium or aluminum.

Figs. 8A - 8C depict an example of how a drug-eluting stent can break down under physiological conditions to release the therapeutic agent. Fig. 9 depicts an exemplary therapeutic agent release profile for the stent of Figs. 8A-8C. Fig. 8A depicts an exemplary embodiment of a drug-eluting stent having layers of bioerodible metal 16, a layer of a therapeutic agent 14, and dispersed phases of therapeutic agent(s) 14 on a structural member 30 of a stent. Figs. 8B and 8C further depict an exemplar process of how the bioerodible metal in such a stent could break down within a patient's body to release one or more therapeutic agents. As shown in Fig. 8B, initially the outer bioerodible metal layer erodes to expose the therapeutic agent layer 14 to the environment of a patent's body. The therapeutic agent in the layer of therapeutic agent 14 can be released over a period of time as the therapeutic agent dissolves or breaks free from the remainder of the stent. The layer of therapeutic agent 14 can include a pure therapeutic agent, a mixture of therapeutic agents, or a therapeutic agent including inactive ingredients. The therapeutic agent layer 14 can include a polymer, be polymer-free, or be in the form of a ceramic. Fig. 8C depicts the lower layer of bioerodible metal 16 further eroding to release the discrete phases of therapeutic agent(s) 14 embedded within the bioerodible metal 16. The therapeutic agent(s) can dissolve at a faster rate than the bioerodible metal 16, leaving cavities 19 in the outer surface of the magnesium coating. The bioerodible metals for each layer can be selected to determine the rate of erosion. For example, the first layer can be pure magnesium and the second layer can be an alloy of magnesium.

Fig. 9 depicts an exemplary drug release schedule. The exemplary drug release schedule is for a stent having magnesium as the bioerodible metal and Taxus SR as the entrapped therapeutic agent. As shown, the surface drug deposit may allow for an immediate elution of a therapeutic agent without the need for the erosion of a bioerodible metal. The magnesium then erodes to allow for a second period of a greater amount of eluting drug between the 50 and 100 days period. By using multiple layers of bioerodible metal (of varying compositions and/or thicknesses) and of therapeutic agents (of varying compositions and thicknesses), a variety of drug regimens with varying release profiles can be created. Changes in the alloy composition modulate the time required for a complete biocorrosion, ranging from 1 day to 2 months. Bioerodible metal coatings could be comprised of several metals known to be implantable and degradable, such as magnesium, iron, zirconium, and/or alloys thereof. These coatings could be abluminal or encapsulate the stent. Furthermore, as described in relationship to Figs. 6A-6C, the use of two or more bioerodible metals of different electronegativities in electrical contact with each other can allow for the selective delay in erosion of the more electronegative of the bioerodible metals.

The various layers and/or discrete phases of therapeutic agent(s) and bioerodible metal(s) can be deposited onto a metal structural member 30 of a stent in a variety of ways. One method for producing coatings of combinations of therapeutic agents and bioerodible metals is to use Cold Gas Dynamic Spray ("CGDS"). CGDS accelerates a pressurized carrier gas through a de Laval type nozzle to supersonic velocities. Metal powders or particles are mixed with the gas to accelerate the particles to supersonic velocities. When the powders or particles impact a surface, their momentum deforms and micro-welds them into the surface, producing a bonded metal film with compressive stresses. The metal powders or particles can vary in material and size to produce different coating features. CGDS operating parameters may be adjusted to control the compaction of the particle and/or powders on the substrate surface to control the amount of porosity; the greater the compaction, the less porosity. CGDS spray processes are described in U.S. Patent 5,302,414 ("Alkhomev et al.") and in U.S. Patent 6,139,913 ("Van Steenkiste,"). CGDS is useful because CGDS processes can allow for the creation of metal coatings at lower temperatures. Some therapeutic agents can be sensitive to high temperatures, which may alter or destroy them.

CGDS spray techniques can be used to create a variety of different bioerodible metal and therapeutic agent arrangements. CGDS can be used to coat a layer of bioerodible metal 16 onto a drug coating and/or discrete drug deposits 14 deposited on a stent structural member 30 to produce an arrangement similar to those depicted in the figures. Additional layers of bioerodible metal 16 could also be added by this technique. CGDS can also be used to deposit therapeutic agents in layers or discrete phases, but therapeutic agents can also be deposited by other methods.

Drug can also be deposited by other techniques, and is not limited to the CGDS spray techniques. These techniques can include dipping, spraying, roll coating, and ink-jetting. These processes can be controlled to give full layers or discreet regions. Codeposition techniques, for depositing both the therapeutic agent and the metals together in one layer, can include pulsed laser deposition and sol gel techniques. In some embodiments, a solution of the drug can be applied by spraying, dipping, roll-coating, and by inkjet printing. For example, a drug solution can be immersed into a porous structures by dip-coating for a couple of hours.

Fig. 10 depicts a CGDS process of producing a drug-eluting stent coating including bioerodible phases 16 and therapeutic agent phases 14 and 19. As shown in Fig. 10, the therapeutic agent phase 34 and the bioerodible metal phases 36 can be concurrently deposited using CGDS. The therapeutic agents deposited by the CGDS process can be in a ceramic form or within a polymer. The process can also be used to deposit a variety of different bioerodible metals in combination with non-bioerodible metals. By controlling the relative amount of therapeutic agent verses the amount of metal and the sizes of the deposits, the process can ensure that at least some of the therapeutic agent deposits will require the erosion of at least a portion of the bioerodible metal 16 under physiological conditions before the therapeutic agent is released. This process can be completed by depositing a final layer of bioerodible metal to overlie all of the therapeutic agent deposits. Alternatively, some of the therapeutic agent deposits 19 can be left with an exposed surface to create a stent that will immediately release drug once implanted within a patient's body.

CGDS processes can also be used to deposit non-bioerodible metals. For example, a non-bioerodible metal could be concurrently deposited along with therapeutic agent and bioerodible metal to produce a structure similar to that shown in Fig. 7A.

The terms "therapeutic agent", "pharmaceutically active agent", "pharmaceutically active material", "pharmaceutically active ingredient", "drug" and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. By small organic molecule is meant an organic molecule having 50 or fewer carbon atoms, and fewer than 100 non-hydrogen atoms in total.

Exemplary non-genetic therapeutic agents for use in conjunction with the presently disclosed endoprostheses an include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) alpha receptor antagonist (such as doxazosin, Tamsulosin) and beta receptor agonists (such as dobutamine, salmeterol), beta receptor antagonist (such as atenolol, metaprolol, butoxamine), angiotensin-II receptor antagonists (such as losartan, valsartan, irbesartan, candesartan and telmisartan), and antispasmodic drugs (such as oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, and (y) human apolioproteins (e.g., AI, AII, AIII, AIV, AV, etc.).

Specific examples of non-genetic therapeutic agents include paclitaxel, (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2) , as well as derivatives of the forgoing, among others.

Exemplary genetic therapeutic agents for use in conjunction with the presently disclosed endoprostheses include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in conjunction with the presently disclosed endoprostheses include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the presently disclosed endoprostheses and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (I) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-βpathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin, macrolide antibiotics such as erythromycin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Further additional therapeutic agents include immunosuppressents such as sirolimus and antibiotics such as macrolide antibiotics, evorolimus, zotarolimus, tacrolimus, picrolimus, and Tacrolimus for the presently disclosed endoprostheses are also disclosed in U.S. Patent No. 5,733,925.

A wide range of therapeutic agent loadings can be used in conjunction with the presently disclosed endoprostheses, with the therapeutically effective amount being readily determined by those of ordinary skill in the art and ultimately depending, for example, upon the condition to be treated, the age, sex and condition of the patient, the nature of the therapeutic agent, the nature of the ceramic region(s), and/or the nature of the endoprosthesis, among other factors.

Stent 20 can be of any desired shape and size (e.g., coronary stents, aortic stents, peripheral vascular stents, gastrointestinal stents, urology stents, and neurology stents). Depending on the application, the stent can have a diameter of between, for example, 1 mm to 46 mm. In certain embodiments, a coronary stent can have an expanded diameter of from 2 mm to 6 mm. In some embodiments, a peripheral stent can have an expanded diameter of from 5 mm to 24 mm. In certain embodiments, a gastrointestinal and/or urology stent can have an expanded diameter of from 6 mm to about 30 mm. In some embodiments, a neurology stent can have an expanded diameter of from about 1 mm to about 12 mm. An Abdominal Aortic Aneurysm (AAA) stent and a Thoracic Aortic Aneurysm (TAA) stent can have a diameter from about 20 mm to about 46 mm.

In use, a stent can be used, e.g., delivered and expanded, using a catheter delivery system. Catheter systems are described in, for example, Wang U.S. 5,195,969, Hamlin U.S. 5,270,086, and Raeder-Devens, U.S. 6,726,712. Stents and stent delivery are also exemplified by the Sentinol^{®} system, available from Boston Scientific Scimed, Maple Grove, MN.

In some embodiments, stents can also be a part of a covered stent or a stent-graft. In other embodiments, a stent can include and/or be attached to a biocompatible, nonporous or semi-porous polymer matrix made of polytetrafluoroethylene (PTFE), expanded PTFE, polyethylene, urethane, or polypropylene.

In some embodiments, stents can be formed by fabricating a wire having a therapeutic agent and a bioerodible metal, and knitting and/or weaving the wire into a tubular member.

### Example

A stent made of stainless steel (e.g. the BSC Liberte^{®} stent) is sprayed with pure Paclitaxol. The pure Paclitaxol is sprayed using a conventional gas-assisted nozzle. The stent is only allowed a brief time in the spray plume to produce discrete drug spots as shown in Fig. 2. The stent is then placed inside a vacuum chamber where it is rotated in front of a stream of high velocity nanoparticles produced by a Mantis nanoparticle generator (Mantis Ltd, Thame, UK). A fine horizontal grid of wires is used as a mask close to the front of the stent. The grid has wires of sizes approx 10-20microns diameter and a spacing of >2x the diameter. After deposition of a bioerodible magnesium the grid is moved to mask the magnesium coated regions and unmask the drug areas that have not been coated by magnesium. The grid is attached to a piezoelectric nanopositioner that shifts the grid by a distance equal to half the period of the grid to achieve this masking shift. A bioerodible iron is then deposited on the remaining uncoated area of the stent. The result is circumferential rings of alternate magnesium and iron coated drug spots along the stent struts. The magnesium will bioerode first releasing then underlying drug as a 'burst effect'. The iron will then bioerode at a slower rate and produce a more prolonged drug release.

## Claims

1. A drug-eluting endoprosthesis, comprising a body defined by a plurality of struts, the body defining a flow passage therethrough, at least one strut of the plurality of struts including
- a plurality of discrete deposits (32, 33), and
- a plurality of overlying layers (34, 35, 36, 37) each overlying one of the plurality of discrete deposits (32, 33),
each discrete deposit (32, 33) comprising one or more therapeutic agents, each overlying layer (34, 35, 36, 37) comprising one or more bioerodible metals, wherein the overlying layers (34, 35, 36, 37) erode in a physiological environment to release the one or more therapeutic agents.

2. The drug-eluting endoprosthesis of claim 1, wherein the overlying layers (34, 35, 36, 37) each comprise a bioerodible metal selected from the group consisting of magnesium, zinc, iron, and alloys thereof.

3. The drug-eluting endoprosthesis of claim 1, wherein at least two of the overlying layers comprise different bioerodible metal compositions.

4. The drug-eluting endoprosthesis of claim 3, wherein the at least two overlying layers comprising different bioerodible metal compositions and each overlie discrete deposits comprising therapeutic agents of different compositions.

5. The drug-eluting endoprosthesis of claim 1, wherein at least two of the overlying layers have different thicknesses.

6. The drug-eluting endoprosthesis of claim 1, wherein at least two of the discrete deposits (32, 33) comprise therapeutic agents of different compositions.

7. The drug-eluting endoprosthesis of claim 1. wherein the at least one strut comprises a metal selected from the group consisting of stainless steel, platinum enhanced stainless steel, Co-Cr, nitinol, niobium, tantalum, titanium, iridium, platinum, and combinations and alloys thereof.

8. The drug-elution endoprosthesis of claim 1, wherein the at least one strut comprises a primer layer (31).

9. The drug-eluting endoprosthesis of claim 1, wherein the discrete deposits (32, 33) comprise a ceramic or polymeric carrier.

10. The drug-eluting endoprosthesis of claim 1, wherein the plurality of discrete deposits are on an abluminal side of the strut.

11. The drug-eluting endoprosthesis of claim 1, wherein the endoprosthesis is a stent.

12. The drug-eluting endoprosthesis of claim 8, wherein the primer layer (31) is the same bioerodible metal overlying the therapeutic agent

13. A method for forming a drug-eluting endoprosthesis, the method comprising:
- depositing a plurality of discrete deposits onto at least on strut of body including a plurality of interconnected struts, the discrete deposits each comprising at least one therapeutic agent, the body defining a flow passage therethrough; and
- depositing a plurality of overlying layers so that each of the plurality of overlying layers overlies one discrete deposit, the plurality of overlying layers each comprising a bioerodible metal.

14. The method of claim 13, further comprising:
- applying a primer layer onto the at least one strut;
- depositing the plurality of discrete deposits onto the primer layer.

15. The method of claim 13. wherein at least two of the overlying layers each include different bioerodible metal compositions, different thicknesses, or a combination thereof.

## Patentansprüche

1. Eine Wirkstoff eluierende Endoprothese umfassend einen Körper, der durch eine Vielzahl von Streben definiert wird, wobei der Körper eine Durchflusspassage definiert und zumindest eine Strebe der Vielzahl von Streben beinhaltet
- eine Vielzahl von separaten Ablagerungen (32, 33), und
- eine Vielzahl von überlagernden Schichten (34, 35, 36, 37), jede über einer der Vielzahl von separaten Ablagerungen (32, 33) liegend,
jede separate Ablagerung (32, 33) umfassend eine oder mehrere therapeutische Wirkstoffe,
jede überlagernde Schicht (34, 35, 36, 37) umfassend ein oder mehrere bioerodierbare Metalle, wobei die überlagernden Schichten (34, 35, 36, 37) in einer physiologischen Umgebung erodieren, um den einen oder die mehreren therapeutischen Wirkstoffe freizusetzen.

2. Die Wirkstoff eluierende Endoprothese nach Anspruch 1, wobei jede der überlagernden Schicht (34, 35, 36, 37) ein bioerodierbares Metall ausgewählt aus der Gruppe bestehend aus Magnesium, Zink, Eisen, oder Legierungen davon umfasst.

3. Die Wirkstoff eluierende Endoprothese nach Anspruch 1, wobei zumindest zwei der überlagernden Schichten verschiedene bioerodierbare Metallzusammensetzungen umfassen.

4. Die Wirkstoff eluierende Endoprothese nach Anspruch 3, wobei die zumindest zwei überlagernden Schichten verschiedene bioerodierbare Metallzusammensetzungen umfassen und jede über separaten Ablagerungen umfassend therapeutische Wirkstoffe von unterschiedlicher Zusammensetzung, liegt.

5. Die Wirkstoff eluierende Endoprothese nach Anspruch 1, wobei zumindest zwei der überlagernden Schichten verschiedene Dicken haben.

6. Die Wirkstoff eluierende Endoprothese nach Anspruch 1, wobei zumindest zwei der separaten Ablagerungen (32, 33) therapeutische Wirkstoffe verschiedener Zusammensetzung umfassen.

7. Die Wirkstoff eluierende Endoprothese nach Anspruch 1, wobei die zumindest eine Strebe ein Metall ausgewählt aus der Gruppe bestehend aus Edelstahl, Platin-verstärktem Edelstahl, Co-Cr, Nitinol, Niob, Tantal, Titan, Iridium, Platin, oder Kombinationen oder Legierungen davon umfasst.

8. Die Wirkstoff eluierende Endoprothese nach Anspruch 1, wobei die zumindest eine Strebe eine Primer-Schicht (31) umfasst.

9. Die Wirkstoff eluierende Endoprothese nach Anspruch 1, wobei die separaten Ablagerungen (32, 33) einen keramischen oder polymerischen Träger umfassen.

10. Die Wirkstoff eluierende Endoprothese nach Anspruch 1, wobei die Vielzahl von separaten Ablagerungen auf einer abluminalen Seite der Strebe sind.

11. Die Wirkstoff eluierende Endoprothese nach Anspruch 1, wobei die Endoprothese ein Stent ist.

12. Die Wirkstoff eluierende Endoprothese nach Anspruch 8, wobei die Primer-Schicht (31) das gleiche bioerodierbare Metall wie das über dem therapeutischen Wirkstoff liegende ist.

13. Ein Verfahren zum Herstellen einer Wirkstoff eluierenden Endoprothese, das Verfahren umfassend:
- Aufbringen einer Vielzahl von separaten Ablagerungen auf zumindest einer Strebe eines Körpers beinhaltend eine Vielzahl von miteinander verbundenen Streben, wobei die separaten Ablagerungen jede zumindest einen therapeutischen Wirkstoff umfasst, wobei der Körper eine Durchflusspassage definiert; und
- Aufbringen einer Vielzahl von überlagernden Schichten so, dass jede der Vielzahl von überlagernden Schichten eine separate Ablagerung überlagert, wobei jede der Mehrzahl von überlagernden Schichten ein bioerodierbares Metall umfasst.

14. Das Verfahren nach Anspruch 13, weiter umfassend:
- Anbringen einer Primer-Schicht auf zumindest einer Strebe;
- Aufbringen einer Vielzahl von separaten Ablagerungen auf die Primer-Schicht.

15. Das Verfahren nach Anspruch 13, wobei jede von zumindest zwei der überlagernden Schichten verschiedene bioerodierbare Metallzusammensetzungen, verschiedene Dicken, oder Kombinationen daraus beinhaltet.

## Revendications

1. Endoprothèse élutrice de médicaments, comprenant un corps défini par une pluralité d'entretoises, le corps définissant un passage d'écoulement à travers celui-ci, au moins une entretoise de la pluralité d'entretoises comprenant
- une pluralité de dépôts discrets (32, 33), et
- une pluralité de couches de revêtement (34, 35, 36, 37), chacune recouvrant un dépôt de la pluralité de dépôts discrets (32, 33),
chaque dépôt discret (32, 33) comprenant un ou plusieurs agents thérapeutiques,
chaque couche de revêtement (34, 35, 36, 37) comprenant un ou plusieurs métaux bioérodables, les couches de revêtement (34, 35, 36, 37) s'érodant dans un environnement physiologique pour libérer le ou les agents thérapeutiques.

2. Endoprothèse élutrice de médicaments selon la revendication 1, dans laquelle les couches de revêtement (34, 35, 36, 37) comprennent chacune un métal bioérodable choisi dans le groupe constitué par le magnésium, le zinc, le fer, et les alliages de ceux-ci.

3. Endoprothèse élutrice de médicaments selon la revendication 1, dans laquelle au moins deux des couches de revêtement comprennent des compositions métalliques bioérodables différentes.

4. Endoprothèse élutrice de médicaments selon la revendication 3, dans laquelle les au moins deux couches de revêtement comprenant des compositions métalliques bioérodables différentes recouvrent chacune des dépôts discrets comprenant des agents thérapeutiques de différentes compositions.

5. Endoprothèse élutrice de médicaments selon la revendication 1, dans laquelle au moins deux des couches de revêtement ont des épaisseurs différentes.

6. Endoprothèse élutrice de médicaments selon la revendication 1, dans laquelle au moins deux des dépôts discrets (32, 33) comprennent des agents thérapeutiques de différentes compositions.

7. Endoprothèse élutrice de médicaments selon la revendication 1, dans laquelle l'au moins une entretoise comprend un métal choisi dans le groupe constitué par l'acier inoxydable, l'acier inoxydable amélioré au platine, le Co-Cr, le nitinol, le niobium, le tantale, le titane, l'iridium, le platine, et les combinaisons et alliages de ceux-ci.

8. Endoprothèse élutrice de médicaments selon la revendication 1, dans laquelle l'au moins une entretoise comprend une couche primaire (31).

9. Endoprothèse élutrice de médicaments selon la revendication 1, dans laquelle les dépôts discrets (32, 33) comprennent un support céramique ou polymère.

10. Endoprothèse élutrice de médicaments selon la revendication 1, dans laquelle la pluralité de dépôts discrets se trouve sur un côté abluminal de l'entretoise.

11. Endoprothèse élutrice de médicaments selon la revendication 1, l'endoprothèse étant un stent.

12. Endoprothèse élutrice de médicaments selon la revendication 8, dans laquelle la couche primaire (31) est le même métal bioérodable recouvrant l'agent thérapeutique.

13. Procédé de formation d'une endoprothèse élutrice de médicaments, le procédé comprenant :
- le dépôt d'une pluralité de dépôts discrets sur au moins une entretoise d'un corps comprenant une pluralité d'entretoises interconnectées, les dépôts discrets comprenant chacun au moins un agent thérapeutique, le corps définissant un passage d'écoulement à travers celui-ci ; et
- le dépôt d'une pluralité de couches de revêtement de telle sorte que chaque couche de la pluralité de couches de revêtement recouvre un dépôt discret, la pluralité de couches de revêtement comprenant chacune un métal bioérodable.

14. Procédé selon la revendication 13, comprenant en outre :
- l'application d'une couche primaire sur l'au moins une entretoise ;
- le dépôt de la pluralité de dépôts discrets sur la couche primaire.

15. Procédé selon la revendication 13, dans lequel au moins deux des couches de revêtement ont chacune des compositions métalliques bioérodables différentes, des épaisseurs différentes, ou une combinaison de celles-ci.
